(19) Europäisches Patentamt
European Patent Office
Office européen des brevets

(11) **EP 4 167 187 A1**

(12) **EUROPEAN PATENT APPLICATION**

(43) Date of publication:
**19.04.2023 Bulletin 2023/16**

(21) Application number: **22201168.6**

(22) Date of filing: **12.10.2022**

(51) International Patent Classification (IPC):
***G06T 11/00*** *(2006.01)*

(52) Cooperative Patent Classification (CPC):
**G06T 11/005;** G06T 2211/436

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.10.2021 US 202163254812 P**
**10.10.2022 US 202217962734**

(71) Applicant: **Canon Medical Systems Corporation Tochigi 324-0036 (JP)**

(72) Inventors:
• **HU, Yi**
**Tochigi (JP)**
• **CHANG, Yu-bing**
**Tochigi (JP)**
• **DUTTA, Komal**
**Tochigi (JP)**
• **IWAI, Haruki**
**Tochigi (JP)**
• **HUA, Rui**
**Tochigl (JP)**
• **MANAK, Joseph**
**Tochigi (JP)**

(74) Representative: **Marks & Clerk LLP**
**15 Fetter Lane**
**London EC4A 1BW (GB)**

(54) **X-RAY DIAGNOSTIC APPARATUS AND A MEDICAL INFORMATION PROCESSING METHOD**

(57)     An X-ray diagnostic apparatus (100) according to an embodiment includes processing circuitry (200) configured to improve quality of fourth data corresponding to a fourth number of views that is smaller than a first number of views by inputting the fourth data to a learned model generated by performing machine learning with second data corresponding to a second number of views as input side data, and third data corresponding to a third number of views that is larger than the second number of views as output side data, the second data and the third data being acquired based on first data corresponding to the first number of views. The fourth data is data acquired by tomosynthesis imaging.

FIG.2

FIG.4A

EP 4 167 187 A1

**Description**

FIELD

Embodiments relate to an X-ray diagnostic apparatus and a medical information processing method.

BACKGROUND

Techniques that collect a plurality of pieces of projection data with varying X-ray radiation angles (views) and reconstruct reconstructed image data have been known. For example, in tomosynthesis, a plurality of pieces of projection data are collected by varying a view within a certain angle range. Computed tomography (CT), on the other hand, can collect "360°" worth of projection data.

From the viewpoint of reducing radiation exposure or the like, a narrower angle range over which the view is changed is preferable. For example, tomosynthesis can reconstruct high-quality reconstructed image data by collecting projection data from an angle range of approximately "100°". However, it is also possible to reconstruct reconstructed image data based on projection data collected from an angle range of approximately "60°". When limited-angle (limited angle) imaging with a narrower angle range is used, it is possible to benefit from reduced radiation exposure and shorter imaging time.

From the viewpoint of reducing radiation exposure, it is conceivable to collect projection data for sparse views with a reduced number of times of X-ray radiation, instead of collecting projection data for each of consecutive views within a certain angle range. When such sparse-view imaging is used, the benefits of reduced radiation exposure and cost savings can be achieved.

Imaging methods that reduce the number of views, such as sparse views and limited angles, have various benefits, including reduced radiation exposure, and have been studied in recent years. For example, Patent Literature 1 describes "receiving a sparse-view CT data and reconstructing an image for the sparse-view CT data using a neural network of a learning model satisfying a predetermined frame condition." Other articles such as Non-Patent Literature 1 and Non-Patent Literature 2 also have discussed the use of sparse view data in medical imaging.

Summary of Invention

An X-ray diagnostic apparatus comprises processing circuitry configured to improve quality of fourth data corresponding to a fourth number of views that is smaller than a first number of views by inputting the fourth data to a learned model generated by performing machine learning with second data corresponding to a second number of views as input side data, and third data corresponding to a third number of views that is larger than the second number of views as output side data, the second data and the third data being acquired based on first data corresponding to the first number of views, wherein the fourth data is data acquired by tomosynthesis imaging.

The first data may be projection data of the first number of views, and the second data may be generated by downsampling the first data.

The first data may be projection data of the first number of views, and the second data may be generated by performing forward projection of the second number of views on reconstructed image data reconstructed from the first data.

The X-ray diagnostic apparatus according to claim 1, wherein the fourth data is projection data of the fourth number of views, and the processing circuitry inputs the fourth data to the learned model to generate processed projection data corresponding to a fifth number of views that is larger than the fourth number of views, and performs reconstruction processing on the generated processed projection data to generate reconstructed image data.

The fourth data may be reconstructed image data generated by performing reconstruction processing on projection data of the fourth number of views, and the processing circuitry may generate reconstructed image data of higher quality than quality of the fourth data by inputting the fourth data to the learned model.

The storage circuitry may store, as the learned model, a first learned model that receives input of projection data to improve quality of the projection data and a second learned model that receives input of reconstructed image data to improve quality of the reconstructed image data, and the processing circuitry may input projection data of the fourth number of views to the first learned model to generate processed projection data corresponding to a fifth number of views that is larger than the fourth number of views, perform reconstruction processing on the generated processed projection data to generate processed reconstructed image data, and input the processed reconstructed image data to the second learned model to improve quality of the processed reconstructed image data.

The fourth data may be projection data of the fourth number of views, and the processing circuitry may input the fourth data to the learned model to adjust a parameter related to an iterative reconstruction method, and uses the adjusted parameter to generate reconstructed image data.

The second data may be data corresponding to a sparser sparse view than the third data, or data corresponding to a limited angle with a narrower angle range than the third data.

The learned model may be generated by machine learning using the first data as the third data.

Improving the quality of the fourth data is to cause the fourth data to have the same quality as the quality of the third data.

The fourth data may be projection data of the fourth number of views, and the data obtained by improving the quality of the fourth data may be data of the same number of views as the third number of views.

The reconstructed image data of high quality may be the fourth data with the same quality as the quality of the third data.

Improving the quality of the fourth data may be at least one of noise reduction, artifact reduction, and improved spatial resolution.

The learned model may be generated by using a generative adversarial network (GAN).

The learned model may be generated by using a discriminator network (DNN).

The second data may comprise input image data and the third data may comprise target image data. The learned model may be generated by performing machine learning with the second data as input data and the third data as target data. The learned model may be a generator neural network (GNN). The learned model may be generated by taking the second data as input to a generator neural network (GNN) to produce fake image data. A discriminator network (DNN) is then trained with the fake image data and the third data, using training labels that the fake image data is fake and the third data is real. The DNN is then held fixed while the GNN is trained using a loss function associated with an output of the fixed DNN. This process is repeated in alternating phases to generate a learned GNN.

A medical information processing apparatus comprising processing circuitry, the processing circuitry being configured to:acquire, based on first data corresponding to a first number of views, second data corresponding to a second number of views and third data corresponding to a third number of views that is larger than the second number of views; and perform machine learning with the second data as input side data and the third data as output side data to generate a learned model.

A medical information processing method comprising improving quality of fourth data corresponding to a fourth number of views that is smaller than a first number of views by inputting the fourth data to a learned model generated by performing machine learning with second data corresponding to a second number of views as input side data, and third data corresponding to a third number of views that is larger than the second number of views as output side data, the second and third data being acquired based on first data corresponding to the first number of views.

A medical information processing method comprising: acquiring, based on first data corresponding to a first number of views, second data corresponding to a second number of views and third data corresponding to a third number of views that is larger than the second number of views; and performing machine learning with the second data as input side data and the third data as output side data to generate a learned model.

A medical information processing method comprising improving quality of fourth data corresponding to a fourth number of views that is smaller than the first number of views by inputting the fourth data to the learned model generated according to the above described method.

An X-ray diagnostic apparatus comprising processing circuitry configured to improve quality of fourth data corresponding to a fourth number of views that is smaller than the first number of views by inputting the fourth data to the learned model generated according to the above described method.

BRIEF DESCRIPTION OF THE DRAWINGS

FIG. 1 is a diagram showing an example of a configuration of an X-ray diagnostic apparatus.
FIG. 2 is a diagram showing an example of a projection data acquisition method.
FIG. 3 is a diagram showing an example of a configuration of a medical information processing system.
FIG. 4A is a diagram showing an application example of a learned model.
FIG. 4B is a diagram showing an application example of a learned model.
FIG. 4C is a diagram showing an application example of a learned model.
FIG. 4D is a diagram showing an application example of a learned model.
FIG. 5 is a diagram of a general training process for training a GNN by using a DNN to estimate whether the image generated by the GNN is "real enough" to fool the DNN.
FIGS. 6A-6C are diagrams of additional image datasets used in a general training process for training the GNN of FIG. 5 with different missing bands of data at acquired at different angles.
FIG. 6D is a diagrams of generalized additional image datasets used in a general training process for training the GNN of FIG. 5 in which generally Dense-view /full-angle range sinogram datasets are used with a DNN to teach the GNN to produce enhanced image data from Sparse-view/limited-angle sinogram datasets.

FIG. 7 is a data flow diagram showing a basic operation of a general method of using the trained GNN trained in FIGS. 5-6C to create corrected sinogram data.

FIG. 8A is a combined data flow diagram illustrating an embodiment in which, rather than performing training on sinogram datasets, training is performed on image data after reconstruction.

FIG. 8B is a combined data flow diagram illustrating an embodiment in which, rather than performing training on sinogram datasets, training is performed on image data after reconstruction and image capture information on the images is included in the training of the GNN and DNN so that image capture-specific image generation can be performed.

FIG. 9 is a data flow diagram showing a general method by which training data can be generated for the training processes described herein.

FIG. 10 is a data flow diagram showing a method by which training data can be generated for the training processes described herein by converting existing CT data using a forward projection tool to produce image datasets that can be intentionally degraded (e.g., with simulated noise, blurring or binning) to produce both Simulated Dense-view/large-angle data and Simulated sparse-view/limited-angle data.

FIG. 11 is a combined data flow diagram illustrating an embodiment in which, rather than performing training on only sinogram datasets, training also is performed on image data after reconstruction.

FIG. 12 is a data flow diagram illustrating using plural input images and plural real images simultaneously (e.g., 2 input images at a time and 2 real images at a time) in order to train a more complicated GNN/DNN pair.

FIG. 13 is a data flow diagram illustrating using kernel-based processing of images (e.g., using an exemplary 3x3x3 kernel) in order to train a more complicated GNN/DNN pair.


DETAILED DESCRIPTION

The following describes embodiments of an X-ray diagnostic apparatus, a medical information processing apparatus, and a medical information processing method, with reference to the accompanying drawings.

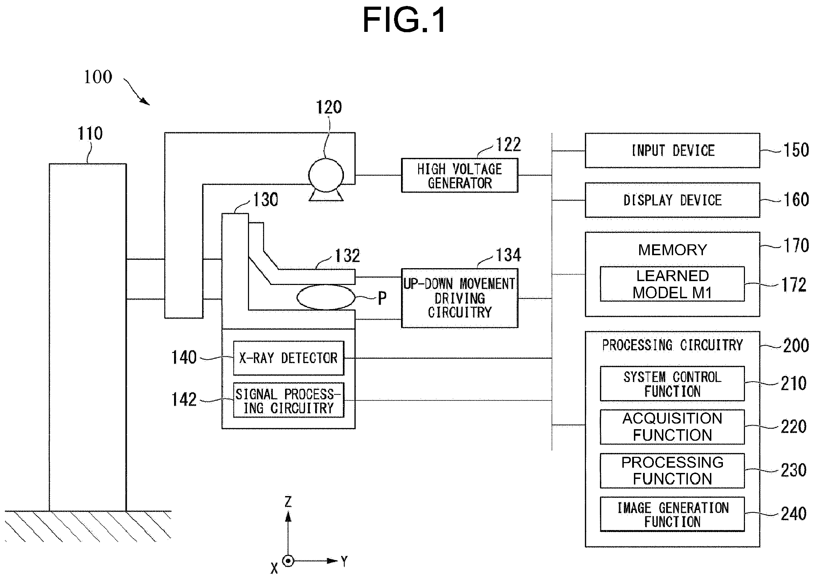

Figure 1 (taken from Figure 17 of U.S. Patent Publication No. 2020/0285902 ("the '902 publication)) is an illustration depicting an imaging environment in which a patient/subject can be imaged. FIG. 1 is a diagram showing an example of a configuration of an X-ray diagnostic apparatus 100 including medical image processing according to the seventh embodiment in the "902 publication". For example, the X-ray diagnostic apparatus 100 may include a stand 110, an X-ray tube 120, a high voltage generator 122, an imaging stand 130, a pressing plate 132, up-down movement driving circuitry 134, an X-ray detector 140, signal processing circuitry 142, an input device 150, a display device 160, and processing circuitry 200.

The stand 110 supports the imaging stand 130, the pressing plate 132, the X-ray detector 140, and the signal processing circuitry 142 such that they can move in the vertical direction (Z direction in FIG. 1).

The X-ray tube 120 generates X-rays using a high voltage supplied from the high voltage generator 122. The X-ray generated by the X-ray tube 120 is radiated to the position (radiation target position) of the breast of a test object P. The X-ray tube 120 can move to draw an arc around the Y axis in FIG. 1 such that a radiation angle for the radiation target position can be changed. The high voltage generator 122 is connected to the X-ray tube 120 and supplies a high voltage for generating X-rays to the X-ray tube 120 according to control of the processing circuitry 200.

The imaging stand 130 is a stand supporting the breast of the test object P. The imaging stand 130 has a supporting surface on which the breast of the test object P is placed. The pressing plate 132 is attached to the upper part of the imaging stand 130 (in the +Z direction in FIG. 1) to face the supporting surface of the imaging stand 130. The pressing plate 132 can move in a direction (Z direction in FIG. 1) in which it becomes far away from or approaches the imaging stand 130 while remaining in a state in which it faces the supporting surface of the imaging stand 130.

The breast of the test object P is placed between the imaging stand 130 and the pressing plate 132. Accordingly, the breast of the test object P is pressed when the pressing plate 132 moves in a direction in which it approaches the imaging stand 130. Therefore, it is possible to obtain a clearer image by thinly stretching and extending the breast of the test object P to reduce overlap of mammary gland.

The up-down movement driving circuitry 134 is connected to the imaging stand 130 and moves the imaging stand 130 up and down according to control of the processing circuitry 200. In addition, the up-down movement driving circuitry 134 is connected to the pressing plate 132 and moves the pressing plate 132 up and down according to control of the processing circuitry 200.

The X-ray diagnostic apparatus 100 can generate a mammographic image such as a mediolateral-oblique (MLO) image or a cranio-caudal (CC) image on the basis of projection data collected by fixing the positions of the imaging stand 130 and the pressing plate 132 in an MLO direction or a CC direction and radiating X-rays in a state a specific radiation angle to the breast of the test object P is maintained.

The X-ray detector 140 is a flat panel detector (FPD), for example. The X-ray detector 140 detects X-rays that have passed through the test object P and converts the X-rays into an electronic signal. The X-ray detector 140 has thin film transistor (TFT) sensor pixels, for example. The X-ray detector 140 stores the electronic signal that is a detection result therein. The signal processing circuitry 142 reads the electronic signal converted by the X-ray detector 140, generates projection data on the basis of the electronic signal and stores the projection data in a memory 170.

The input device 150 receives an input operation of an operator operating the X-ray diagnostic apparatus 100. For example, the input device 150 includes a mouse, a keyboard, buttons, a trackball, a joystick, a touch panel, or the like. The input device 150 converts details of the input operation into electronic signals and outputs the electronic signals to the processing circuitry 200.

The display device 160 display various images generated by the processing circuitry 200. The various images include a graphical user interface (GUI) images for receiving a touch operation of the operator, cross-sectional images generated from tomosynthesis, two-dimensional images, etc.

The memory 170 includes storage circuitry having a non-transitory storage medium such as an HDD, a flash memory or a ROM, and storage circuitry such as a RAM or a register, for example. The memory 170 stores projection data, tomosynthesis, a program executed by the processing circuitry 200, a learned model M1, etc. The learned model M1 may be implemented in such a manner that it is embedded in a program. The memory 170 is an example of a storage unit.

The processing circuitry 200 executes a system control function 210, an acquisition function 220, a processing function 230, and a display control function 240, for example. The processing circuitry 200 realizes these functions by a processor executing programs stored in the memory 170, for example.

The system control function 210 controls the entire X-ray diagnostic apparatus 100. For example, the system control function 210 may generate control signals with respect to the high voltage generator 122, the up-down movement driving circuitry 134, the X-ray detector 140 and the like and outputs the control signals thereto.

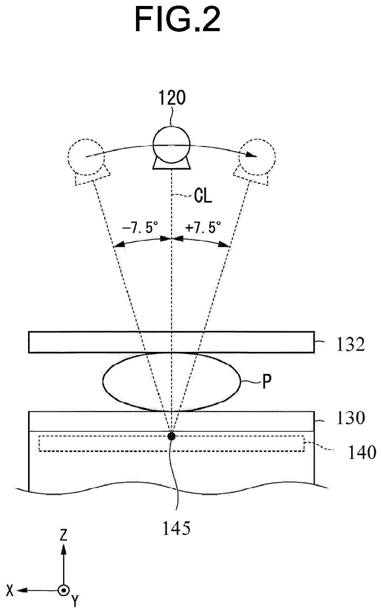

An example of details of control performed by the system control function 210 will be described. FIG. 2 is a diagram showing an example of operation of tomosynthesis imaging performed by the X-ray diagnostic apparatus 100. For example, in an imaging step, the X-ray tube 120 is driven by a driving mechanism which is not shown to move while drawing an arc within an angle range of -7.5° to +7.5° with a position (neutral position) on a normal line CL with respect to a detection surface of the X-ray detector 140, which passes through a center point 145 in the X direction of the detection surface, as a center. The above-described neutral position corresponds to when the X-ray tube 120 is at an angle of 0°. In this process, the X-ray detector 140 and the signal processing circuitry 142 generate 11 pieces of projection data such that, for example, angle intervals with respect to the center point 145 of the x-ray tube 120 are each -1.5°. That is, projection data with the number of views "11" is generated from the angle range of -7.5° to +7.5°. These 11 pieces of projection data include projection data generated when the X-ray tube 120 is on the normal line CL. According to this operation, a plurality of pieces of projection data for tomosynthesis can be acquired.

The acquisition function 220 reads various data from the memory 170 and provides the read data to other functions. For example, the acquisition function 220 performs logarithmic conversion processing, offset correction processing, sensitivity correction processing, beam hardening correction processing on projection data stored in the memory 170 through the signal processing circuitry 142 and provides the corrected projection data to other functions.

The processing function 230 performs reconstruction processing on the projection data on which correction processing has been performed by the image acquisition function 220 to generate a tomosynthesis image. For example, the processing function 230 generates a tomosynthesis image by performing image back projection processing based on reverse radon transformation on a plurality of pieces of projection data. As a specific example of image back projection processing, a filtered back projection method, an iterative reconstruction (IR) method, a shift addition method, and the like are known, and the processing function 230 can employ any method with respect to these specific methods.

The processing function 230 improves the quality of reconstructed image data by inputting datasets associated with tomosynthesis imaging of the test object to the learned model M1. The data input to the learned model M1 may be projection data or reconstructed image data after reconstruction processing. The processing function 230 is an example of a processing unit.

The reconstructed image data may be volume data or one or more tomographic image data. For example, the volume data is 3D data having (x, y, z) information. The tomographic image data is 2D data having (x, y) information. In this case, a plurality of tomographic image data can be generated for each z coordinate.

The display control function 250 generates an image signal for causing the display device 160 to display the tomosynthesis image generated by the processing function 230 and the like and outputs the image signal to the display device 160.

According to the seventh embodiment in the "902 publication", it is possible to cause the X-ray diagnostic apparatus to operate as any medical image processing apparatus of the first to sixth embodiments in the "902 publication".

The X-ray diagnostic apparatus 100 illustrated in FIGS. 1 and 2 is only an example. The specific configuration of the apparatus is not particularly limited as long as it is capable of collecting projection data for a plurality of views. For example, although FIGS. 1 and 2 illustrate a mammography apparatus with the pressing plate 132, the X-ray diagnostic apparatus 100 need not be a mammography apparatus. For example, the X-ray diagnostic apparatus 100 may be an X-ray TV apparatus with a C-arm. In this case, the X-ray diagnostic apparatus 100 holds the X-ray tube 120 by the C-arm, changes the X-ray radiation angle by controlling the rotational motion of the C-arm, and collects projection data for a plurality of views.

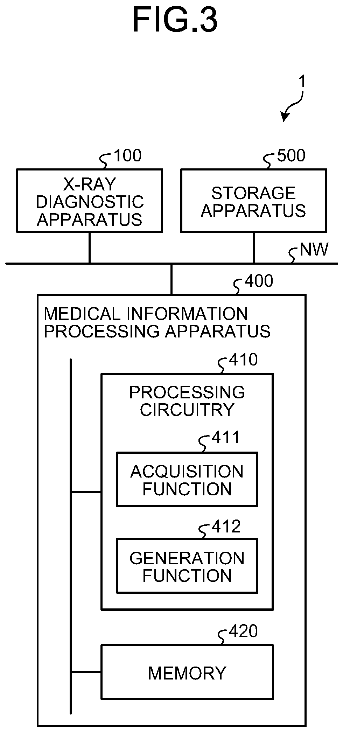

Next, the learned model M1 is explained using FIG. 3. FIG. 3 shows an example of a configuration of a medical information processing system 1. The medical information processing system 1 includes the X-ray diagnostic apparatus 100, a medical information processing apparatus 400, and a storage apparatus 500. For example, the learned model M1 is generated by the medical information processing apparatus 400. The medical information processing apparatus 400 is connected to the X-ray diagnostic apparatus 100 via a network NW.

For example, the medical information processing apparatus 400 is connected to a plurality of modality apparatuses, such as the X-ray diagnostic apparatus 100, and acquires projection data and reconstructed image data collected at the modality devices as learning data for generating the learned model M1. Alternatively, the medical information processing apparatus 400 acquires the projection data and reconstructed image data collected at the modality devices and stored in the storage apparatus 500 as learning data for generating the learned model M1. A specific example of the storage apparatus 500 is a picture archiving and communication system (PACS) server.

The medical information processing apparatus 400 includes, for example, processing circuitry 410 and a memory 420. The processing circuitry 410 performs an acquisition function 411 and a generation function 412. The processing circuitry 410 realizes these functions by a processor executing programs stored in the memory 420, for example. The acquisition function 411 is an example of an acquisition unit. The generation function 412 is an example of a generation unit.

The memory 420 includes storage circuitry having a non-transitory storage medium such as an HDD, a flash memory, or a ROM, and storage circuitry such as a RAM or a register, for example. The memory 420 stores the learned model M1, learning data for generating the learned model M1, and a program executed by the processing circuitry 410.

Specifically, the acquisition function 411 acquires, based on first data corresponding to a first number of views, second data corresponding to a second number of views and third data corresponding to a third number of views that is larger than the second number of views. The generation function 412 performs machine learning with the second data as input side data and the third data as output side data to generate the learned model M1.

The "first data corresponding to the first number of views" is a generic term for the projection data of the first number of views and the reconstructed image data generated by the reconstruction processing for the projection data of the first number of views. The same applies to the "second data corresponding to the second number of views," the "third data corresponding to the third number of views," and the "fourth data corresponding to the fourth number of views" described later.

In the following, the case where the first data is a full-view sinogram collected by an X-ray CT apparatus will be described. That is, the first data may be X-ray data collected by CT. A sinogram is an example of projection data. A full-view sinogram is dense-view data acquired by x-ray exposures the number of which is not reduced at the time of imaging, and is large-angle data collected from an angle range of "360°".

The acquisition function 411 acquires the second and third data based on the first data. Here, the second data is a sparser sparse-view sinogram than the third data is, or a limited-angle sinogram with a narrower angle range than that of the third data.

For example, the acquisition function 411 downsamples the first data to generate the second and third data.

For example, the acquisition function 411 extracts "N=3n" views of the first data as the second data and "N=2n" views as the third data, where "N" is a number assigned to each view of the first data and "n" is a natural number. This allows the acquisition function 411 to generate the second data and the third data with a higher number of views than the second data. In other words, the acquisition function 411 can generate the second data, which is a sparse-view sinogram, and the third data, which is a dense-view sinogram.

To take another example, the acquisition function 411 extracts the views included in some angle range of the "360°" angle range of the first data as the second and third data. For example, the acquisition function 411 extracts as the second data the views included in an angle range of "60°" out of the angle range of "360°" of the first data, and as the third data the views included in an angle range of "100°". The angle range "60°" of the second data is preferably included in the angle range "100°" of the third data. This allows the acquisition function 41 1 to generate the second data and the third data with a higher number of views than the second data. In other words, the acquisition function 411 can generate the second data, which is a limited-angle sinogram, and the third data, which is a large-angle sinogram.

The case of generating the second and third data by downsampling has been described, but the embodiments are not limited thereto. For example, the acquisition function 411 may perform forward projection on the reconstructed image data reconstructed from the first data to generate the second and third data.

The method for reconstructing reconstructed image data from the first data is not particularly limited, and any of the reconstruction methods described above can be employed. The acquisition function 411 may perform AI-based reconstruction, such as deep learning reconstruction (DLR). The first data is dense-view and large-angle data, and high-quality reconstructed image data can be reconstructed even if existing reconstruction methods are employed.

The acquisition function 411 generates the second and third data by performing a plurality of times of forward projection processing with varying projection angles on the reconstructed image data reconstructed from the first data. The second data can be made into a sparse-view sinogram by increasing the interval at which the projection angle is changed, and the third data can be made into a dense-view sinogram by decreasing the interval at which the projection angle is changed. The second data can be made into a limited-angle sinogram by varying the projection angle within a narrow angle range, and the third data can be made into a large-angle sinogram by varying the projection angle within a wide angle range.

The acquisition function 411 may use the first data as it is as the third data. That is, the process of generating the third data can be omitted. The acquisition function 411 acquires a plurality of pairs of the second and third data described above and stores them in the memory 420 as learning data.

The generation function 412 reads the learning data from the memory 420 and performs machine learning with the second data as input side data and the third data as output side data to generate the learned model M1. The learned model M1 can be realized by a neural network, such as a generative adversarial network (GAN) or a discriminator network (DNN).

The learned model M1 generated by the generation function 412 is transmitted to the X-ray diagnostic apparatus 100 via the network NW, for example, and stored in the memory 170. The processing function 230 improves the quality of the fourth data corresponding to the fourth number of views that is smaller than the first number of views by inputting the fourth data to the learned model M1 read from the memory 170. The fourth data is, for example, a sinogram collected from the test object P by the X-ray diagnostic apparatus 100 under control by the system control function 210.

The quality of a sinogram is, for example, the number of views of that sinogram. The quality of reconstructed image data includes, for example, the number of errors such as noise and artifacts, the presence or absence of blurring, and spatial resolution. That is, reconstructed image data quality is image quality. The number of views of a sinogram is closely related to the image quality of the reconstructed image data generated based on the sinogram. For example, it is known that, when a sparse-view sinogram is subjected to reconstruction processing by a conventional method, streak artifacts can occur in the reconstructed image data. From the above, the number of views of the sinogram and the image quality of the reconstructed image data are not specifically distinguished, but are simply described as data quality as well.

For example, if the second data is a sinogram and the third data is a sinogram with more views than those of the second data, the generation function 412 generates a learned model Mil by machine learning using the second and third data. The learned model M11 is functionalized to receive input of a sinogram and increase the number of views of the input sinogram. That is, the learned model M11 is functionalized to improve the quality of the input sinograms.

For example, if the second data is a sparse-view sinogram and the third data is a dense-view sinogram, the learned model Mil can be functionalized to generate a dense-view sinogram by complementing between views in the second data. That is, the learned model M11 is functionalized to perform interpolation. If the second data is a limited-angle sinogram and the third data is a large-angle sinogram, the learned model M11 is functionalized to extend the angle range in the second data to generate a large-angle sinogram. That is, the learned model M11 is functionalized to perform extrapolation.

By performing machine learning using both sparse-view/dense-view learning data and limited-angle/large-angle learning data, the learned model Mil can increase the number of views of the input sinogram by performing at least one of interpolation and extrapolation, regardless of whether the input sinogram is a sparse-view sinogram, a limited-angle sinogram, or both.

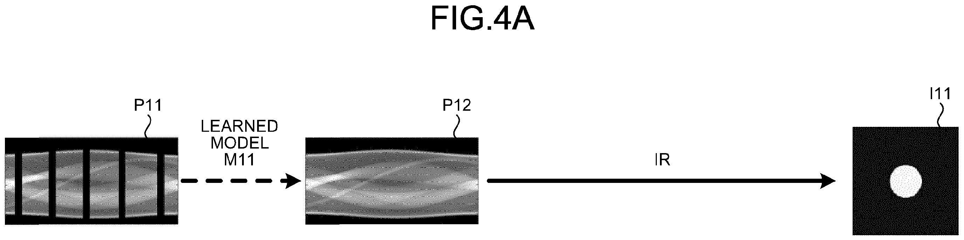

An example of processing by the processing function 230 is illustrated in FIG. 4A. A sinogram P11 is an example of the fourth data, which is, for example, clinical data collected from the test object P by the X-ray diagnostic apparatus 100. As shown in FIG. 4A, the sinogram P11 is the sparse-view data collected by decreasing the number of times of X-ray radiation. For example, the number of views of the sinogram P11 (the fourth number of views) corresponds to the second number of views of the second data used to generate the learned model M11. The processing function 230 can input the sinogram P11 to the learned model Mil and have it interpolated to generate a dense-view sinogram P12. The sinogram P12 is an example of processed projection data corresponding to a fifth number of views that is larger than the fourth number of views. That is, the processing function 230 can improve the quality of the fourth data by inputting the fourth data to the learned model M11.

For example, improving the quality of the fourth data with the learned model Mil is to cause the fourth data to have the same quality as the quality of the third data that is the output side data when the learned model M11 is generated. For example, the data obtained by improving the quality of the fourth data (sinogram P12) is data of the same number of views as the third number of views.

Furthermore, the processing function 230 performs reconstruction processing on the sinogram P12 to generate reconstructed image data I11. For example, the processing function 230 reconstructs the reconstructed image data I11 using an IR method. The sinogram P12 is dense-view data that has been interpolated by the learned model M11, allowing reconstruction of high-quality reconstructed image data 111. That is, the processing function 230 can improve the quality of the reconstructed image data I11 based on the sinogram P11 with a small number of views by the process described above. In FIG. 4A, the sparse-view sinogram P11 is shown as an example of the fourth data, but the same can apply to limited-angle sinograms.

In the preceding description, the case of learning the learned model M1 using sinograms as learning data has been described, but reconstructed image data may also be used as learning data. That is, the second and third data may be reconstructed image data. In this case, the second data is the reconstructed image data generated by the reconstruction processing for the projection data of the second number of views, and the third data is the reconstructed image data generated by the reconstruction processing for the projection data of the third number of views.

For example, the acquisition function 411 in the medical information processing apparatus 400 first acquires the first data. The first data is, for example, a full-view sinogram collected by an X-ray CT apparatus.

The acquisition function 411 then downsamples the first data to generate the second and third data. Specifically, the acquisition function 411 extracts a sinogram of the second number of views from the first data and performs reconstruction processing on the extracted sinogram to generate the second data. The acquisition function 411 also extracts from the first data a sinogram of the third number of views that is larger than the second number of views, and performs reconstruction processing on the extracted sinogram to generate the third data. Here, the third number of views may be the same as the first number of views or smaller than the first number of views. The sinogram of the second number of views extracted from the first data may be a sparse-view sinogram, a limited-angle sinogram, or a sparse-view and limited-angle sinogram. The acquisition function 411 may also use the reconstructed image data reconstructed from the first data as it is as the third data.

Alternatively, the acquisition function 411 performs forward projection on the reconstructed image data reconstructed from the first data to generate the second and third data. Specifically, the acquisition function 411 performs forward projection processing for the second number of views on the reconstructed image data reconstructed from the first data to generate a sinogram for the second number of views, and performs reconstruction processing on the generated sinogram to generate the second data. The acquisition function 411 performs forward projection processing for the third number of views that is larger than the second number of views, on the reconstructed image data reconstructed from the first data to generate a sinogram of the third number of views, and performs reconstruction processing on the generated sinogram to generate the third data. The sinogram of the second number of views generated from the first data by the forward projection processing may be a sparse-view sinogram, a limited-angle sinogram, or a sparse-view and limited-angle sinogram. The acquisition function 411 may also use the reconstructed image data reconstructed from the first data as it is as the third data.

The generation function 412 performs machine learning with the second data as input side data and the third data as output side data to generate the learned model M1. Hereafter, the learned model M1 generated using the second and third data, which is the reconstructed image data, as learning data is described as a learned model M12. The learned model M12 is functionalized to receive input of reconstructed image data and improve the quality of the input reconstructed image data.

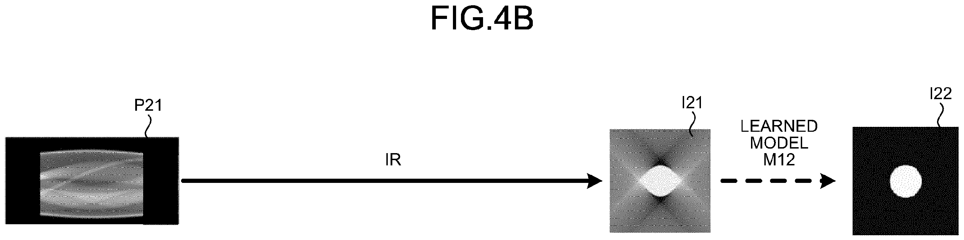

An example of processing by the processing function 230 using the learned model M12 is shown in FIG. 4B. A sinogram P21 is an example of the fourth data, which is, for example, clinical data collected from the test object P by the X-ray diagnostic apparatus 100. In FIG. 4B, the sinogram P21 is limited-angle data taken over a narrow angle range.

The processing function 230 performs reconstruction processing on the sinogram P21 to generate reconstructed image data 121. For example, the processing function 230 reconstructs the reconstructed image data 121 using an IR method. The processing function 230 also inputs the reconstructed image data 121 to the learned model M12 to generate reconstructed image data 122 of higher quality than that of the reconstructed image data 121. For example, the reconstructed image data 122 of high quality is the fourth data with the same quality as the quality of the third data. For example, improving the quality of the fourth data is at least one of noise reduction, artifact reduction, and improved spatial resolution. That is, the processing function 230 can improve the quality of the reconstructed image data 122 based on the sinogram P21 with a small number of views. In FIG. 4B, the limited-angle sinogram P21 is shown as an example of the fourth data, but the same can apply to sparse-view sinograms.

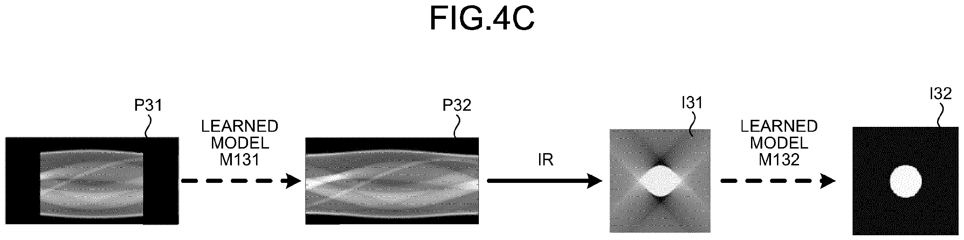

A modification of processing by the processing function 230 is shown in FIG. 4C. A sinogram P31 is an example of the fourth data, which is, for example, clinical data collected from the test object P by the X-ray diagnostic apparatus 100. In FIG. 4C, the sinogram P31 is limited-angle data taken over a narrow angle range.

A learned model M131 shown in FIG. 4C, similarly to the learned model M11 shown in FIG. 4A, is functionalized to receive input of a sinogram and increase the number of views of the input sinogram. A learned model M132, similarly to the learned model M12 shown in FIG. 4B, is functionalized to receive input of reconstructed image data and improve the quality of the input reconstructed image data. The learned model M131 is an example of a first learned model. The learned model M132 is an example of a second learned model.

First, the processing function 230 inputs the sinogram P31 to the learned model M131 and have it extrapolated to generate a large-angle sinogram P32. The processing function 230 then reconstructs reconstructed image data 131 from the sinogram P32. For example, the processing function 230 reconstructs the reconstructed image data 131 using an IR method. Next, the processing function 230 inputs the reconstructed image data 131 to the learned model M132 to generate reconstructed image data 132 of higher quality than that of the reconstructed image data 131.

That is, the processing function 230 inputs projection data of the fourth number of views to the first learned model to generate processed projection data corresponding to the fifth number of views that is larger than the fourth number of views, performs reconstruction processing on the generated processed projection data to generate processed reconstructed image data, and inputs the processed reconstructed image data to the second learned model to improve the quality of the input processed reconstructed image data. This allows the processing function 230 to improve the quality of the reconstructed image data 132 based on the sinogram P31 with a small number of views. In FIG. 4C, the limited-angle sinogram P31 is shown as an example of the fourth data, but the same can apply to sparse-view sinograms.

Note that, the reconstructed image data may be input to the learned model all at once, or may be input sequentially. For example, the processing function 230 collectively inputs volume data or a plurality of tomographic image data to the trained model. For example, the processing function 230 sequentially inputs one or a plurality of tomographic image data to the learned model. In this case, at least one piece of data output from the trained model configures the processed reconstructed image data.

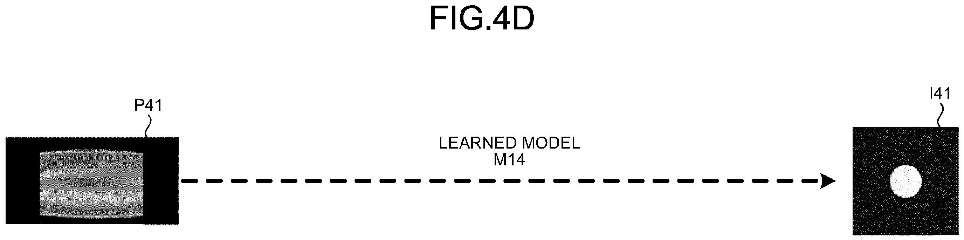

In the preceding description, the learned model M1 that outputs the same type of data as the input data has been described. That is, the learned models Mil and M131, which receive input of projection data and output processed projection data, and the learned models M12 and M132, which receive input of reconstructed image data and output processed reconstructed image data, have been described. However, the embodiments are not limited thereto. A learned model M14, which receives input of projection data and outputs processed reconstructed image data, is described below using FIG. 4D.

For example, the acquisition function 411 in the medical information processing apparatus 400 first acquires a full-view sinogram collected by an X-ray CT apparatus as the first data. Next, the acquisition function 411 generates from the first data, the second data, which is a sinogram, and the third data, which is reconstructed image data.

For example, the acquisition function 411 downsamples the first data to generate the second and third data. Specifically, the acquisition function 411 extracts, as the second data, a sinogram of the second number of views from the first data. The acquisition function 411 also extracts from the first data a sinogram of the third number of views that is larger than the second number of views, and performs reconstruction processing on the extracted sinogram to generate the third data. The sinogram of the sinogram of the second number of views extracted from the first data may be a sparse-view sinogram, a limited-angle sinogram, or a sparse-view and limited-angle sinogram. The acquisition function 411 may also use the reconstructed image data reconstructed from the first data as it is as the third data.

Alternatively, the acquisition function 411 performs forward projection on the reconstructed image data reconstructed from the first data to generate the second and third data. Specifically, the acquisition function 411 performs forward projection processing of the second number of views on the reconstructed image data reconstructed from the first data to generate a sinogram of the second number of views as the second data. The acquisition function 411 performs forward projection processing for the third number of views that is larger than the second number of views, on the reconstructed image data reconstructed from the first data to generate a sinogram of the third number of views, and performs reconstruction processing on the generated sinogram to generate the third data. The sinogram of the second number of views generated from the first data by the forward projection processing may be a sparse-view sinogram, a limited-angle sinogram, or a sparse-view and limited-angle sinogram. The acquisition function 411 may also use the reconstructed image data reconstructed from the first data as it is as the third data.

The generation function 412 performs machine learning with the second data as input side data and the third data as output side data to generate the learned model M14. For example, the learned model M14 is functionalized to adjust parameters related to an IR method.

Here, the parameters related to the IR method may be reconstruction parameters in the IR method or parameters of the processing related to the IR method. For example, the IR method repeatedly updates reconstructed image data to improve quality, and thus the degree to which the pre-updated data is used and the update speed are reconstruction parameters. The IR method involves a seed volume that is the target of the first update. A method is known that applies AI at the seed volume generation stage and uses a seed volume that has been leapfrogged to an appropriate state (hereinafter referred to as "leapfrogging"), rather than simply generating a seed volume using an FBP method or the like. By performing the IR method using the seed volume from the leapfrogging, it is possible to acquire reconstructed image data closer to ground truth without being bound by the local maximum while maintaining data fidelity. The leapfrogging is an example of processing related to an IR method.

The processing function 230 generates reconstructed image data I41 by inputting a sinogram P41 to the learned model M14. Specifically, the learned model M14 receives input of the sinogram P41, adjusts reconstruction parameters and the parameters of the leapfrogging, and generates reconstructed image data I41 using the adjusted parameters.

It is also possible to have AI learn to generate reconstructed image data directly from input of a sinogram without using the IR method. However, it is known that there are cases where non-existent organs or the like are included in reconstructed image data when the entire reconstruction processing is entrusted to AI. In contrast, the learned model M14 described above can improve the quality of reconstructed image data while maintaining data fidelity by using a combination of the IR method and AI.

As described above, the X-ray diagnostic apparatus 100 of the embodiments includes: the memory 170 configured to store the learned model M1 generated by performing machine learning with the second data corresponding to the second number of views as input side data, and the third data corresponding to the third number of views that is larger than the second number of views as output side data, the second and third data being acquired based on the first data corresponding to the first number of views; and the processing function 230 configured to improve the quality of the fourth data corresponding to the fourth number of views that is smaller than the first number of views by inputting the fourth data to the learned model M1. This allows the X-ray diagnostic apparatus 100 to improve the quality of reconstructed image data based on projection data with a small number of views.

In other words, if the quality of reconstructed image data is assured by the above processing, it is possible to reduce the number of times of X-ray radiation and collect sparse-view data in a clinical setting. This allows for various benefits such as reducing the amount of radiation exposure to the test object P and shortening the imaging time.

For example, in breast cancer screening, digital breast tomosynthesis (DBT) can provide 3D information. However, replacing mammography with DBT usually increases radiation exposure. Here, if the processing described above allows sparse-view data collection to be performed, then mammography can be replaced with DBT while avoiding increased radiation exposure.

For example, in vascular interventional surgery, a contrast agent is used to acquire vascular images, and if the amount of contrast agent is large, it may affect a kidney of a test object. Here, if the processing described above enables sparse-view data collection, it will be possible to shorten the imaging time and acquire a wide range of vascular images using less contrast agent.

In an X-ray detector that detects X-rays, signals are read out every time X-rays are radiated. The shorter the period of X-ray radiation, the faster the X-ray detector needs to be driven, and X-ray detectors that can be driven at high speeds are expensive. In contrast, when sparse-view data collection is used, the speed at which the X-ray detector is driven is reduced. In other words, when sparse-view data collection is used, expensive X-ray detectors are no longer needed, which reduces costs.

Alternatively, if the quality of reconstructed image data is assured by the above processing, it is possible to narrow the angle range of imaging and collect limited-angle data in a clinical setting. In this case as well, the same benefits can be acquired, such as reducing the amount of radiation exposure to the test object P and shortening the imaging time.

In addition, the angle range for tomosynthesis imaging is often limited, even if it can be changed in the settings. For example, the upper limit of the angle range in tomosynthesis is approximately "100°". In contrast, if learning has been performed using full-view data from an X-ray CT apparatus as the third data, limited-angle data collected over an angle range of "100°" can be input to the learned model M1 to acquire data equivalent to an angle range of "360°".

Here, the AI for improving the quality of data taken by tomosynthesis can also be generated by machine learning using pairs of low-quality tomosynthesis images and high-quality tomosynthesis images. However, such learning data, specifically for high-quality tomosynthesis images, is difficult to acquire and may be insufficient. In mammography and X-ray TV apparatuses, a radiated X-ray beam is usually conical in shape. This shape increases computational complexity and makes it more difficult to acquire high-quality reconstructed image data.

On the other hand, in the embodiments described above, the learned model M1 can be generated by acquiring learning data (second and third data) from the first data collected by the X-ray CT apparatus to improve the quality of the fourth data taken by tomosynthesis. When the first data is collected by an X-ray CT apparatus, it is not limited to the case of full scan, but even "180° + fan angle" data by half scan, for example, is large-angle projection data as compared with data taken by tomosynthesis, and high-quality reconstructed image data can be relatively easily reconstructed. Furthermore, it is possible to generate a plurality of pairs of the second and third data from the first data in single imaging. Thus, according to the embodiments described above, it is possible to acquire sufficient learning data.

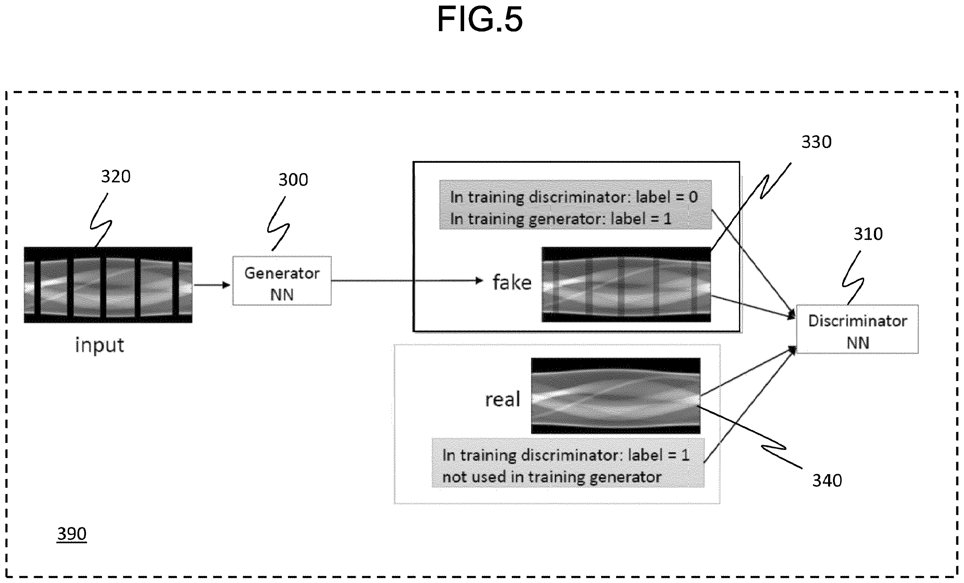

As described above, in the medical information processing system 1 of the embodiments, the extension of the data can be performed by training an untrained neural network using imaging data to produce the learned model M1 capable of extending the acquired data to address at least one of the sparsity of the data and the limited angle range of the data. An example of such a system is shown according to a training process 390 in FIG. 5. In general, a generative neural network (GNN) 300 works in conjunction with a Discriminator Neural Network (DNN) 310 to train the GNN 300 to create realistic data to extend the acquired data to correct for missing data. To do so, a series of input training image datasets 320 are input to the GNN 300 to produce a "fake" image dataset that has been extended to have additional data. Although the image dataset 320 is depicted as being actual image data for easier visual understanding, it should be understood that image datasets 320 and all other "images" described in the following can be either sinogram datasets or image domain datasets without departing from the meaning of "image data." Such a configuration is different than the use of many discriminator networks where inputs to the discriminators are a class (e.g., 0 for cat and 1 for dog). Here, the discriminator receives whole or partial image datasets as part of the inputs to the DNN.

As shown in FIG. 5, the original training image dataset 320 is missing certain data (represented by vertical black bars instead of image data). The "fake" (or "generated") image dataset 330 that is created is shown to have portions of the black bars filled in with data generated by the GNN 300. The DNN 310 is then trained with the fake image dataset 330 and a real image dataset 340. (As used herein, the real image dataset may be referred to as the target image dataset.) For example, as shown in FIG. 5, a real image dataset 340 is utilized that is similar to the input image dataset 320 but which includes additional data as compared to the input image dataset 320 for the same area. (As would be appreciated by those of skill in the art, the real/target image dataset itself may be an artificially generated dataset but with more data than the fake image dataset as is discussed herein with respect to the difference between sparse view data and/or limited angle data versus dense-view/full-angle data.) During this training, the DNN is told (using training labels) that the fake image dataset 330 is fake (by using a training label of 0) and the real image dataset 340 is real (using the training label 1). By using such a technique, it may be possible to address potential instabilities that may exist if, instead, a single set of imaging parameters was used to train a network but the use conditions did not match the training conditions.

In general, this process is repeated in alternating phases where (1) the GNN 300 is held fixed while the DNN 310 is trained, and (2) the DNN 310 is held fixed while the GNN 300 is trained. In the phases where the GNN 300 is held fixed and the DNN 310 is trained, the parameters of the DNN 310 are updated by minimizing the loss function DNNioss of the DNN 310. One such loss function is defined as in equation (1) below:

$$DNN_{loss} = GAN_{loss}(DNN(GNN(input\_image)), \textbf{\textit{0}}) + GAN_{loss}(DNN(real\_image), 1),$$

where $GAN_{loss}(DNN(I1), R/F)$ represents a loss function associated with an output of the DNN 310 when the DNN 310 is given an Image $I1$ and a label $R/F$ indicating real=1 or fake = 0. Exemplary GAN loss functions include, but are not limited to, is binary cross entropy (BCE) functions and Wasserstein loss functions. GNN(input_image) represents the result of applying the input image to the GNN 300. This enables the DNN to learn to discriminate between real and fake images because the labels for the real and fake images are correct. As would be understood by those of skill in the art, alternatively a function can be selected which is to maximized instead, but in either case, a loss function is used that is to be optimized to produce the most effective DNN.

In the phases where the DNN 310 is held fixed and the GNN 300 is trained, the parameters of the GNN 310 are updated by minimizing the loss function GNNioss of the GNN 300. One such loss function is defined as in equation (2) below:

$$GNN_{loss} = GAN_{loss}(DNN(GNN(input\_image)), \textbf{\textit{1}}) +$$

$$alpha * MSE_{loss}(GNN(input\_image), real\_image),$$

where $GAN_{loss}(DNN(I1), R/F)$ is a loss function associated with an output of the currently fixed DNN 310 when the DNN 310 is given an Image $I1$ and a label $R/F$ indicating real=1 and fake = 0. Exemplary GAN loss functions include, but are not limited to, is binary cross entropy (BCE) functions and Wasserstein loss functions. In embodiments where inputs are not a single image (e.g., in environments using multiple-channel image data), another term can be added to minimize the difference across slices to reduce flickering.

Also, $MSE_{loss}$ is an image comparison loss function such as a pixel-by-pixel loss function (e.g., mean squared error or mean absolute error). Here, the $GAN_{loss}$ function is intentionally provided the incorrect label (i.e., real) for whether the image generated by the GNN 300 from the input image (i.e., GNN(input_image)) is real or fake. By giving the DNN 310 the incorrect label by telling it the results from GNN are real, the parameters in G will be updated in such a way as to produce an image that can result in getting a discrimination of "1" after it goes through the DNN, which means that the resulting image is more realistic. As would be understood by those of skill in the art, alternatively a function can be selected which is to maximized instead, but in either case, a loss function is used that is to be optimized to produce the most effective GNN. In addition, the image comparison loss function can be implemented to emphasizing the missing areas can be added to weight higher towards areas of data missing from the original input image. For example, the GNNloss function could instead be implemented as in equation (3) below:

$$GNN_{loss} = GAN_{loss}(DNN(GNN(input\_image)), 1) +$$

$$alpha * MSE_{loss}(Mask(GNN(input\_image)), Mask(real\_image)),$$

where Mask() blacks out the regions that have original data so that the mask focuses the difference analysis on only the part that is newly generated data as compared to the original input image. Given that the GAN utilizes the information related to the real image (not as an input but as part of the process of calculating the MSE), the real image information is made available to the GNN training process as noted in the figures via the use of the dashed line to the GNN.

It is possible to utilize techniques described herein with various sparse-view and limited-angle acquisition scenarios using GAN to perform (1) Sinogram domain correction for handling different angle acquisition scenarios and (2) Image domain correction for handling different cone-beam angle and fan-angle scenarios. To achieve the training datasets, it is possible to perform both real data acquisition and to generate simulated sparse-view and limited-angle acquisition using CT datasets with added system corruption and/or data loss.

Although the network being trained is illustrated as being a GAN, one of ordinary skill in the art will understand that other neural networks (e.g., supervised learning networks) similarly can be trained and used once trained.

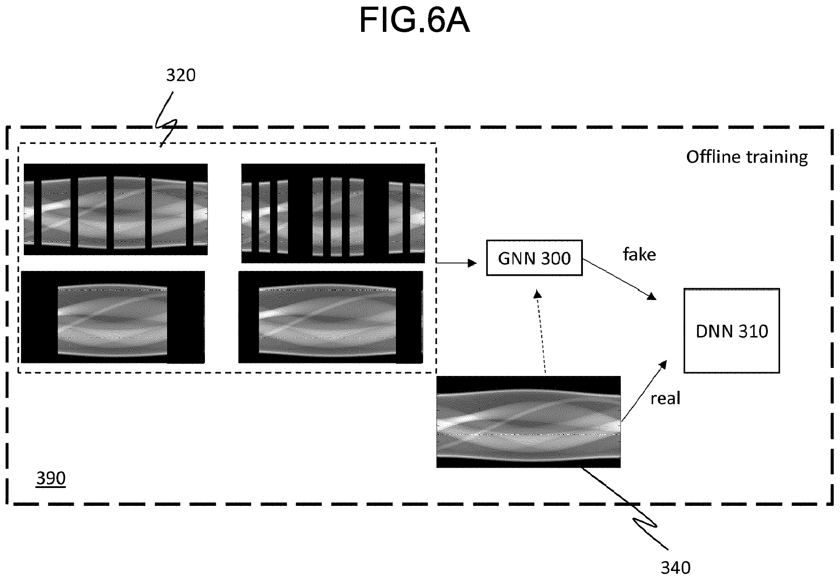

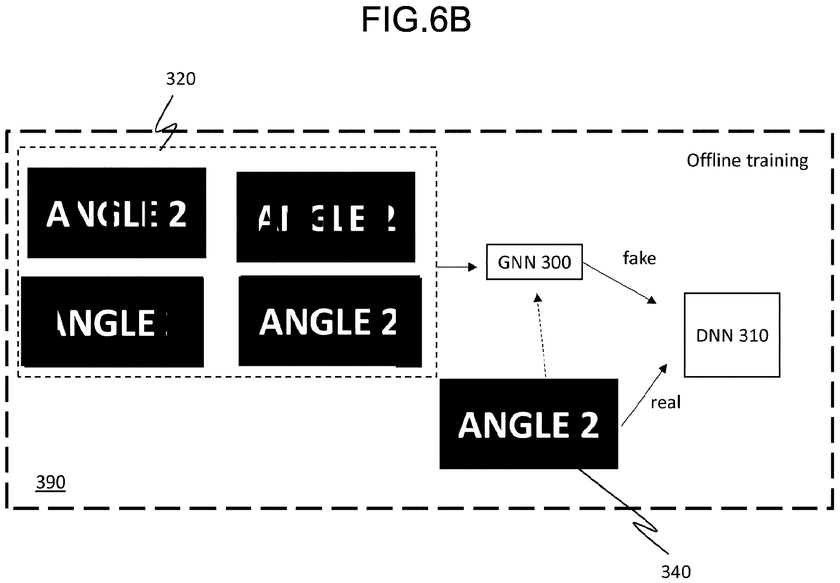

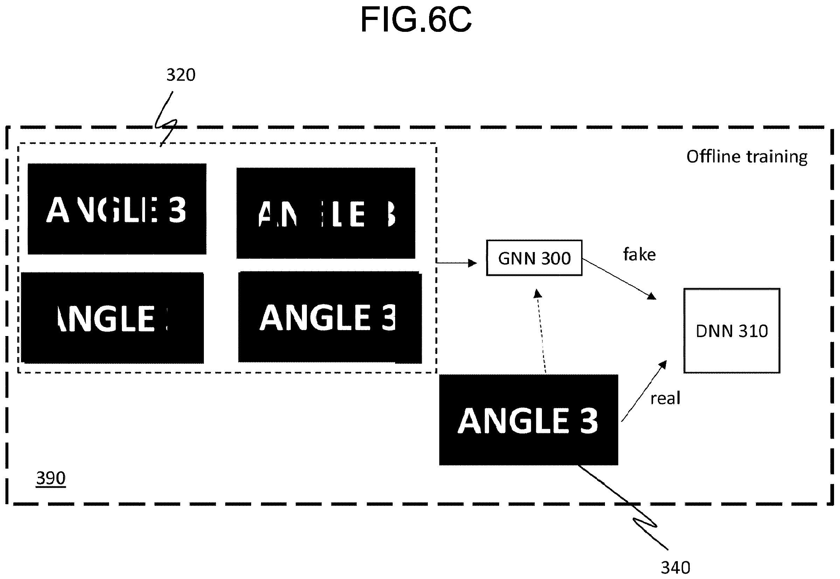

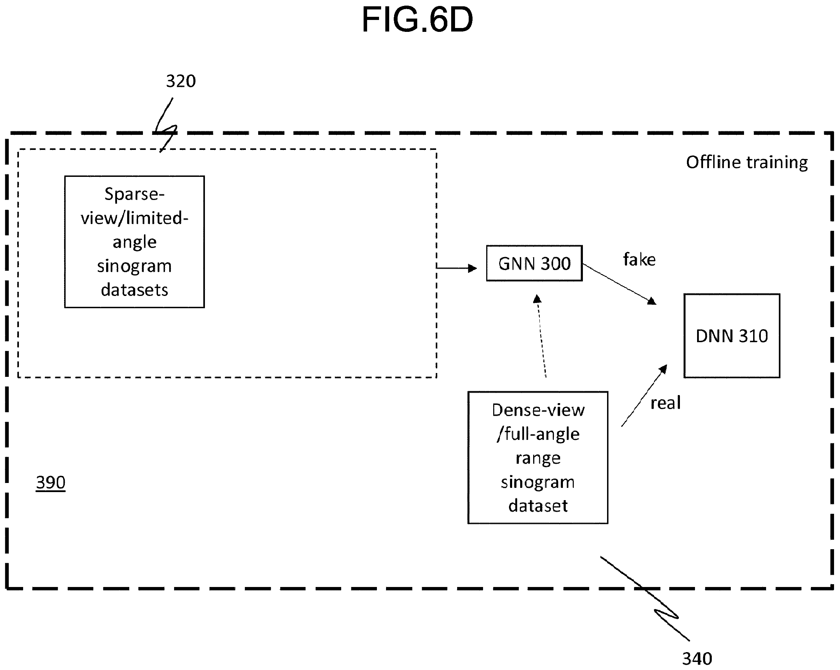

As shown in FIGS. 6A-6C, a variety of image datasets can be used to train the GNN 300. Those of ordinary skill in the art will understand that many sets of image datasets 320 and corresponding real image datasets 340 can and generally should be used to produce the GNN 300. The image datasets 320 among FIGS. 6A-6C are illustrated as (a) having bands of missing data that are narrower or wider, (b) having greater or fewer numbers of bands, and (c) being cropped at different locations. This diversity of input image datasets 320 increases the ability of the GNN 300 to learn to generate meaningful extended in the presence of a greater number of missing data situations. Further, one of ordinary skill will also understand that the image datasets of FIG. 6A are intended to connote having been taken under different image conditions (e.g., cone-beam and fan angles, sparsity rates, and exposure conditions), and that the different image conditions can be tracked on a pixel-by-pixel basis. As will be explained later, in alternate embodiments, the imaging conditions may further be applied to the GNN and DNN to provide imaging condition-specific data generation.

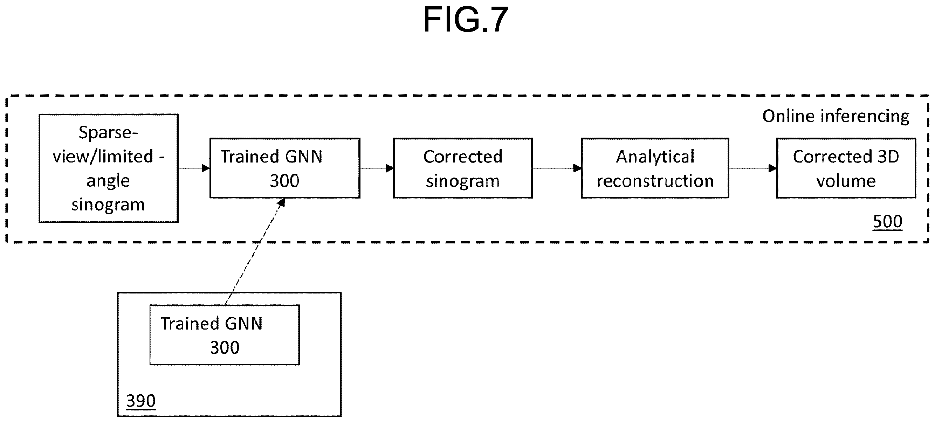

FIG. 7 is a data flow diagram showing a basic operation of a general method of using the trained GNN trained in FIGS. 3-4C to create corrected sinogram data. As illustrated in FIG. 7, the training process 390 of FIGS. 3-4C results in a trained GNN 300 that is used in the process 500 of data generation. Later acquired sparse-view/limited angle sinogram datasets can be applied to the trained GNN 300 to produce corrected sinogram data. The corrected sinogram data can then be processed using conventional analytical reconstruction techniques to produce corrected 3D reconstructed image datasets.

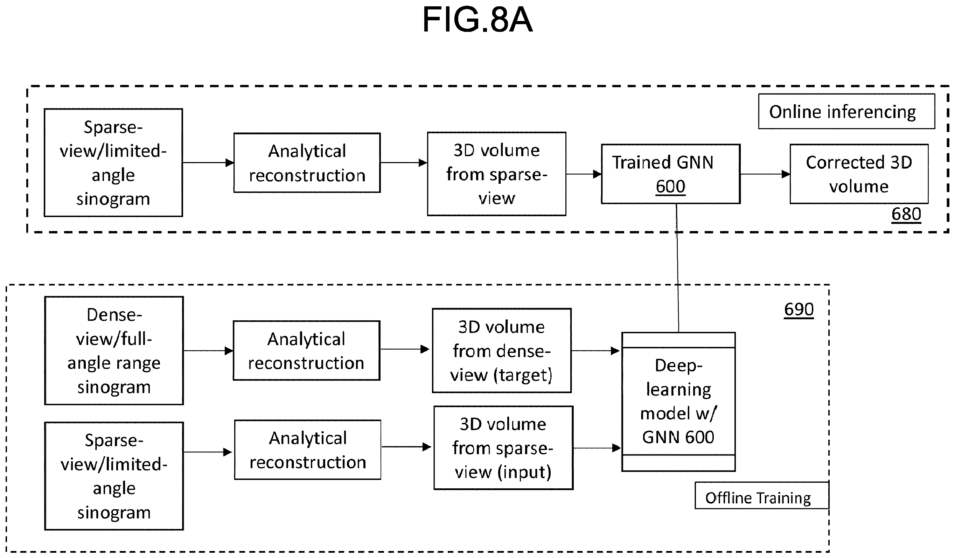

FIG. 8A is a combined data flow diagram illustrating an embodiment in which, rather than performing training on sinogram datasets, training is performed on image data after reconstruction. In the illustrated embodiment, the Dense-view/full-angle range sinogram datasets and the Sparse-view/limited-angle sinogram datasets are subjected to the same analytical reconstruction processes to produce (1) 3D reconstructed image datasets from the Dense-view/full-angle range sinogram datasets and (2) 3D reconstructed image datasets from the Sparse-view/limited-angle sinogram datasets.

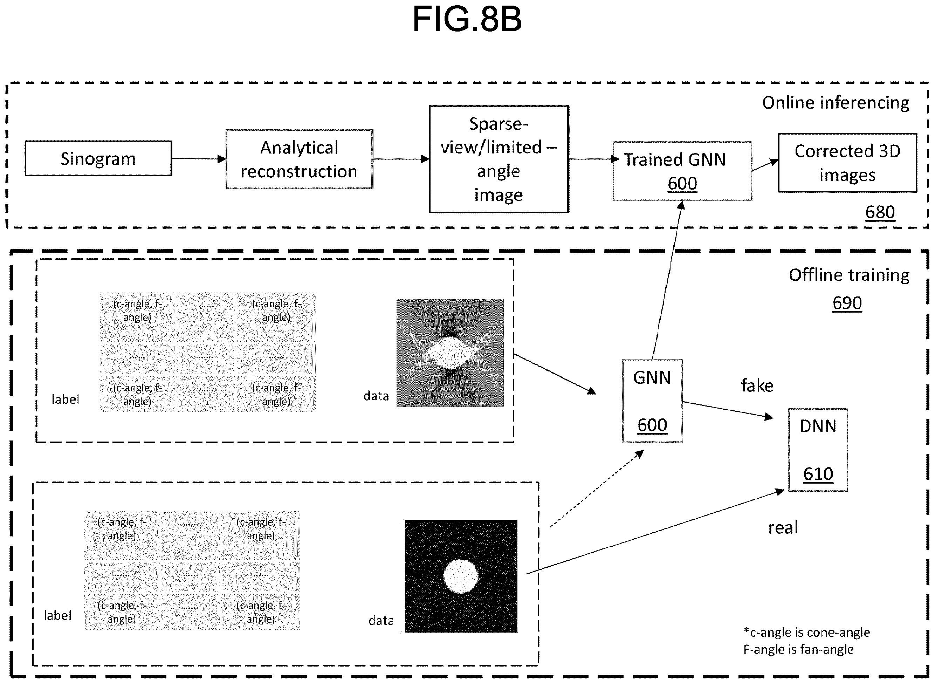

FIG. 8B is a combined data flow diagram illustrating an embodiment in which, rather than performing training on sinogram datasets, training is performed on image data after reconstruction and image capture information on the images is included in the training of the image-based GNN 600 and the image-based DNN 610 so that image capture-specific image generation can be performed. For example, pitch information (i.e., it indicates the angle interval of the X-ray projection, and how sparse/dense the data is), the cone angle and/or the fan angle can be included along with the image dataset. Also, view angle range information can also be included along with the image dataset. Here, the view angle range indicates the range the X-ray tube 120 moves, and during the movement the X-rays are emitted (intermittently or continuously) from the X-ray source. For example, the view angle information can be provided as angle information like "from -7.5 degree to +7.5 degree" as illustrated in Fig. 2. Additional parameters related to exposure conditions (kVp, mAs ) may be included as well as can conditions about a subject being scanned, such as height, weight, ethnicity, and body mass index (BMI).

By including such information, artefacts that are specific to the Sparse-view/limited-angle datasets that do not appear in the dense-view/full-angle range datasets can be corrected by the GNN 600. As illustrated in FIGS. 8A and 8B, the 3D volume-based training process 690 results in a trained GNN 300 that is used in the process 680 of data generation. Later acquired sparse-view/limited angle sinogram datasets can be applied to the trained image-based GNN 600 along with the image capture information noted above, including, but not limited to including pitch information and the view angle range information, about how the acquired sparse-view/limited-angle sinogram dataset was acquired to produce corrected image data. The corrected image data can then be processed using conventional analytical reconstruction techniques to produce corrected 3D reconstructed image datasets. Also, the pitch information or view angle information described above may be included in the sparse view/limited angle sinogram data set. In this case, the actual acquired sparse-view/limited-angle sinogram datasets can be applied to the trained sinogram-based GNN along with the image capture information, including pitch information and the view angle range information, on how the acquired sinogram dataset was acquired to generate corrected sinogram data.

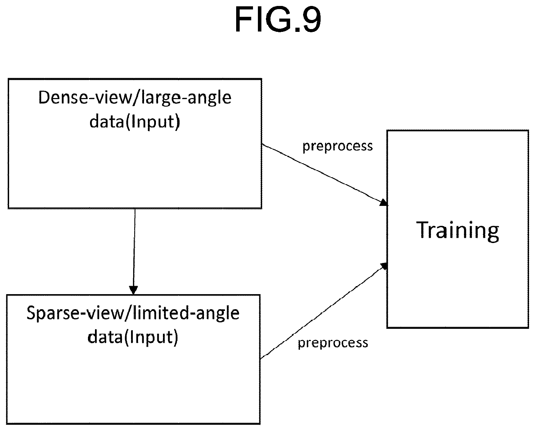

As discussed above, a number of image datasets are used in the training process. FIG. 9 is a data flow diagram showing one method by which training datasets can be generated for the training processes described herein. In the illustrated method, an initial dataset including dense-view/large-angle data is acquired (e.g., from a series of tomosynthesis scan), and the initial dataset is reduced or culled to produce a Sparse-view/limited-angle data corresponding to the imaging device that is intended to be used with the trained GNN. The training can be performed in the sinogram domain or in the image domain after reconstruction.

In addition, it is possible to obtain imaging data from two different imaging devices and directly compare them as opposed to creating a forward projection tool that provides an estimated conversion from one imaging environment to another. For example, a same phantom can be imaged in a CT imaging environment and in a tomosynthesis environment and then direct correlation of data can be used where possible. The phantom can be imaged under a number of imaging conditions, and one or more models can be created from the acquired data. In one such embodiment, image registration is performed on the imaging data from the CT imaging environment to align it with the imaging data from the tomosynthesis environment before the two datasets are used in the generation of the GNN. In one such configuration, the phantom is embedded with internal markers to aid in the automatic registration of the image datasets from the two different imaging environments.

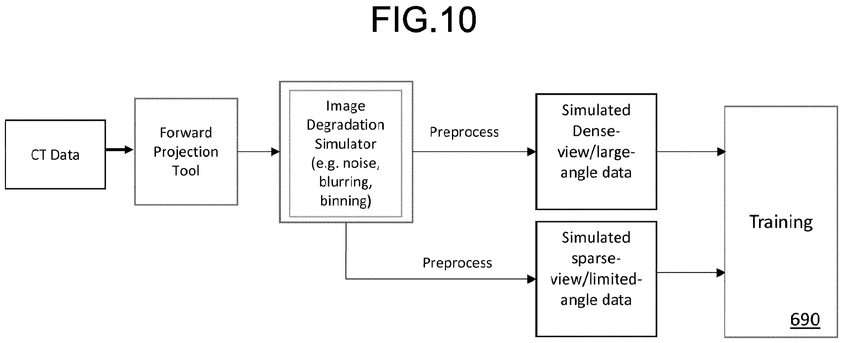

Given that the training can be performed in some embodiments in the image domain, FIG. 10 illustrates a data flow diagram showing a method by which image-based training data can be generated for the training processes described herein by converting existing CT data using a forward projection tool to produce an image dataset that can be intentionally degraded (e.g., with simulated noise, blurring or binning) to produce both Simulated Dense-view/large-angle data and Simulated sparse-view/limited-angle data that can be used in the training process 690.

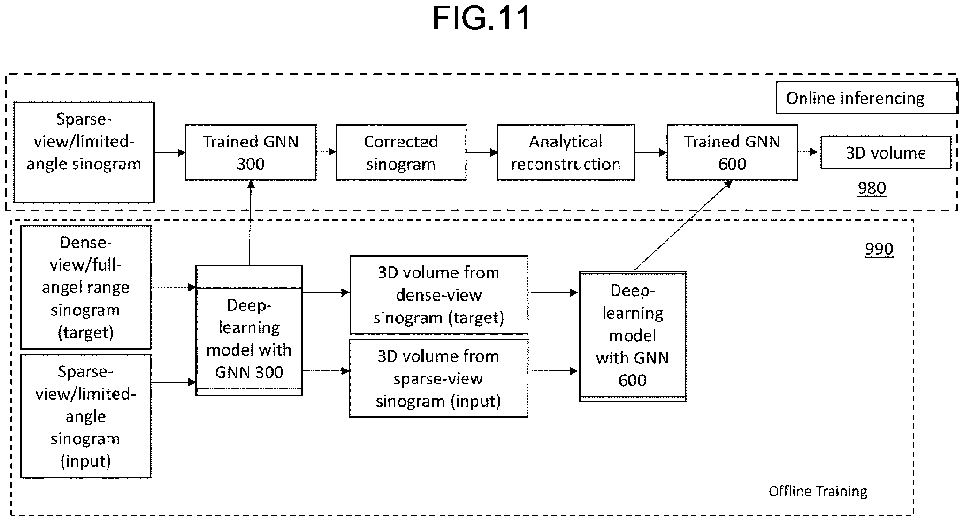

FIG. 11 is a combined data flow diagram illustrating an embodiment in which, rather than performing training on only sinogram datasets, training also is performed on image data after reconstruction so that image capture-specific image generation can be performed. As shown therein, two deep learning models are used to create two GNNs 300 and 600 which are used to produce corrected data in the sinogram and image domains, respectively.

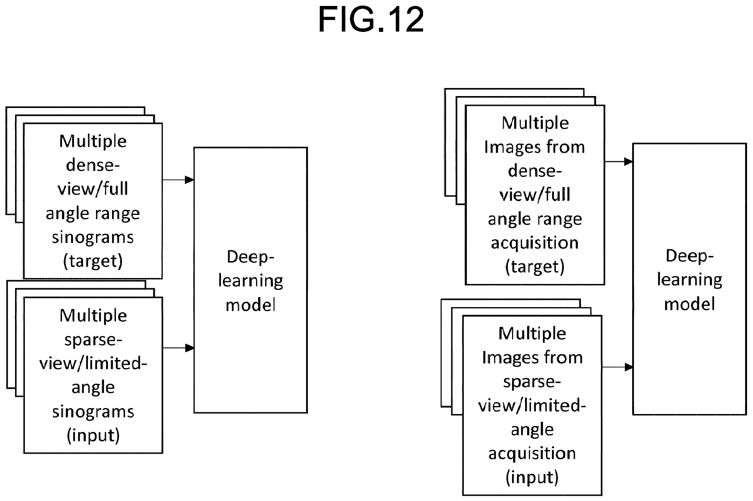

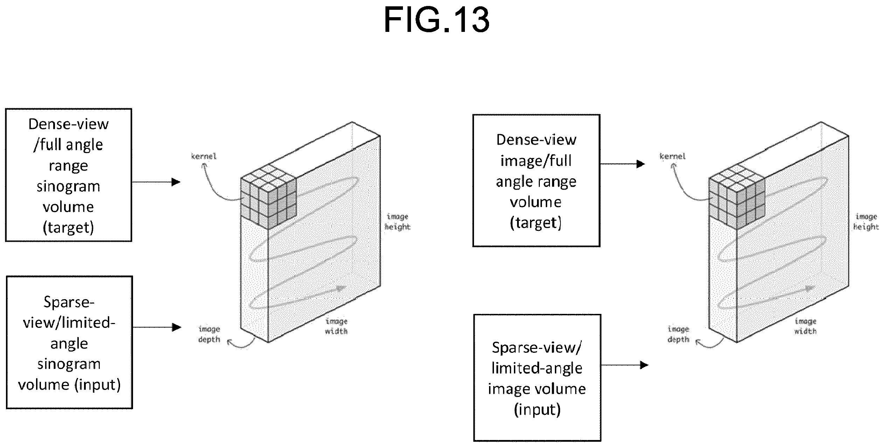

FIG. 12 is a data flow diagram illustrating using plural input images and plural real images simultaneously (e.g., 2 input images at a time and 2 real images at a time) in order to train a more complicated GNN/DNN pair. For example, two adjacent slices can be used simultaneously to help address the realistic generation of data in a 3D model. Such a processing can be extended in three dimensions generally with the use of kernel-based processing. For example, FIG. 13 is a data flow diagram illustrating using kernel-based processing of images (e.g., using an exemplary 3x3x3 kernel) in order to train a more complicated GNN/DNN pair. As would be understood by those of skill in the art, other sized and shaped kernels also are possible, such as 3x6x6 or 3x6x9.

Although a number of configurations herein have been described in which the network being trained is a GAN, one of ordinary skill in the art will understand that other neural networks (e.g., supervised learning networks) similarly can be trained and used once trained. Those networks include both the networks described here that receive only image inputs as well as those that receive addition information (e.g., imaging condition information and other information) in addition to image inputs.

In the embodiments described above, the case in which the second and third data based on the first data are used as learning data is described, but additional learning data may be used. For example, if a plurality of times of tomosynthesis imaging is performed on the same test object and a plurality of pieces of data with different numbers of views are collected, these may be used as additional learning data for machine learning.

In the embodiments described above, the processing of generating the learned model M1 is described as being performed by the processing circuitry 410 of the medical information processing apparatus 400, but the embodiments are not limited thereto. For example, the processing circuitry 200 of the X-ray diagnostic apparatus 100 may include functions equivalent to the acquisition function 411 and the generation function 412 to generate the learned model M1.

In the embodiments described above, the processing of inputting the fourth data to the learned model M1 is described as being performed by the processing circuitry 200 of the X-ray diagnostic apparatus 100, but the embodiments are not limited thereto. For example, the processing circuitry 410 of the medical information processing apparatus 400 may include a function equivalent to the processing function 230 to acquire, via the network NW, the fourth data collected by the X-ray diagnostic apparatus 100 and input it to the learned model M1.

In the embodiments described above, the case in which the first data is collected by an X-ray CT apparatus is described, but the embodiments are not limited thereto. For example, it is possible to achieve the embodiments described above using, as the first data, data taken by tomosynthesis in a mammography apparatus or X-ray TV apparatus. That is, it is possible to generate the second and third data from the first data regardless of the first number of views, and if the number of views differs between the second and third data, this can be learned by the learned model M1 as a difference to achieve the embodiments described above. For example, the first data and the second data may be X-ray data collected by tomosynthesis imaging.

Other types of modality apparatuses for acquiring the first and fourth data can be modified as desired. Although description has been given on diagnostic radiology apparatuses such as X-ray CT apparatuses and X-ray diagnostic apparatuses, the same can apply to other types of modality apparatuses (e.g., MRI, etc.) as well.

In the X-ray diagnostic apparatus 100 shown in FIG. 1, each processing function is stored in the memory 170 in the form of a program executable by a computer. Similarly, in the medical information processing apparatus 400 shown in FIG. 3, each processing function is stored in the memory 420 in the form of a program executable by a computer. These processing circuitry are processors that read programs from a memory and execute them to implement functions corresponding to the respective programs. In other words, the processing circuitry in the state where each program is read out has the function corresponding to the program read out. In each of the above embodiments, the case in which each processing function is implemented by a single processing circuitry is shown, but the embodiments are not limited thereto. For example, processing circuitry may be configured by a combination of a plurality of independent processors, each processor executing a program to implement a processing function. Each processing function of the processing circuitry may be distributed or integrated into a single or a plurality of processing circuitry as appropriate.

The term "processor" used in the above description refers, for example, to circuitry such as a central processing unit (CPU), a graphics processing unit (GPU), or an application-specific integrated circuit (ASIC) or a programmable logic device (for example, a simple programmable logic device (SPLD), a complex programmable logic device (CPLD), and a field programmable gate array (FPGA)). The processor reads and executes a program stored in the memory to implement a function.

In each of the above embodiments, a single memory is described as storing programs corresponding to respective processing functions. However, a plurality of memories may be distributed and disposed, and the processing circuitry may read the programs from respective individual memories. Instead of storing a program in a memory, the program may be incorporated directly into circuitry of the processor. In this case, the processor reads and executes the program incorporated in the circuitry to implement the function.

Each component of the apparatuses in the above embodiments is a functional concept and does not necessarily have to be physically configured as shown in the drawings. In other words, the specific form of distribution and integration of the apparatuses is not limited to those shown in the drawings, but can be configured by functionally or physically distributing and integrating all or some of the apparatuses in any desired unit depending on various loads and usage conditions. Furthermore, all or some of the processing functions performed by each apparatus may be implemented by a CPU and a program analyzed and executed by the CPU, or may be implemented as wired logic hardware.

The medical information processing method described in the embodiments above can be implemented by executing a pre-prepared program on a computer such as a personal computer or workstation. This program can be distributed via the Internet or other networks. The control program can also be recorded on a non-transitory recording medium readable by a computer, such as a hard disk, a flexible disk (FD), a CD-ROM, an MO, or a DVD, and executed by being read from the recording medium by the computer.

According to at least one embodiment described above, the quality of reconstructed image data based on projection data with a small number of views can be improved.

Although some embodiments of the invention have been described, these embodiments are presented as examples and are not intended to limit the scope of the invention. These embodiments can be implemented in various other forms, and various omissions, substitutions, and changes can be made without departing from the gist of the invention. These embodiments and modifications thereof are included within the scope of the invention described in the claims and equivalents thereof as well as within the scope and gist of the invention.

With respect to the above embodiments, the following notes are disclosed as aspects and selective features of the invention.

(Note 1)

An X-ray diagnostic apparatus comprising:

a memory configured to store a learned model generated by performing machine learning with second data corresponding to a second number of views as input side data, and third data corresponding to a third number of views that is larger than the second number of views as output side data, the second data and the third data being acquired based on first data corresponding to a first number of views; and
processing circuitry configured to improve quality of fourth data corresponding to a fourth number of views that is smaller than the first number of views by inputting the fourth data to the learned model.

(Note 2)

The first data may be projection data of the first number of views, and
the second data may be generated by downsampling the first data.

(Note 3)

The first data may be projection data of the first number of views, and

the second data may be generated by performing forward projection of the second number of views on reconstructed image data reconstructed from the first data.

(Note 4)

The X-ray diagnostic apparatus according to claim 1, wherein

the fourth data is projection data of the fourth number of views, and
the processing circuitry inputs the fourth data to the learned model to generate processed projection data corresponding to a fifth number of views that is larger than the fourth number of views, and performs reconstruction processing on the generated processed projection data to generate reconstructed image data.

(Note 5)

The fourth data may be reconstructed image data generated by performing reconstruction processing on projection data of the fourth number of views, and
the processing circuitry may generate reconstructed image data of higher quality than quality of the fourth data by inputting the fourth data to the learned model.

(Note 6)

The storage circuitry may store, as the learned model, a first learned model that receives input of projection data to improve quality of the projection data and a second learned model that receives input of reconstructed image data to improve quality of the reconstructed image data, and
the processing circuitry may input projection data of the fourth number of views to the first learned model to generate processed projection data corresponding to a fifth number of views that is larger than the fourth number of views, perform reconstruction processing on the generated processed projection data to generate processed reconstructed image data, and input the processed reconstructed image data to the second learned model to improve quality of the processed reconstructed image data.

(Note 7)

The fourth data may be projection data of the fourth number of views, and
the processing circuitry may input the fourth data to the learned model to adjust a parameter related to an iterative reconstruction method, and uses the adjusted parameter to generate reconstructed image data.

(Note 8)

The second data may be data corresponding to a sparser sparse view than the third data, or data corresponding to a limited angle with a narrower angle range than the third data.

(Note 9)

The learned model may be generated by machine learning using the first data as the third data.

(Note 10)

Improving the quality of the fourth data is to cause the fourth data to have the same quality as the quality of the third data.

(Note 11)

The fourth data may be projection data of the fourth number of views, and
the data obtained by improving the quality of the fourth data may be data of the same number of views as the third number of views.

(Note 12)

The reconstructed image data of high quality may be the fourth data with the same quality as the quality of the third data.

(Note 13)

Improving the quality of the fourth data may be at least one of noise reduction, artifact reduction, and improved spatial resolution.

(Note 14)

The learned model may be generated by using a generative adversarial network (GAN).

(Note 15)

The learned model may be generated by using a discriminator network (DNN).

(Note 16)

A medical information processing apparatus comprising processing circuitry, the processing circuitry being configured to:

acquire, based on first data corresponding to a first number of views, second data corresponding to a second number of views and third data corresponding to a third number of views that is larger than the second number of views; and perform machine learning with the second data as input side data and the third data as output side data to generate a learned model.

(Note 17)

A medical information processing method comprising improving quality of fourth data corresponding to a fourth number of views that is smaller than a first number of views by inputting the fourth data to a learned model generated by performing machine learning with second data corresponding to a second number of views as input side data, and third data corresponding to a third number of views that is larger than the second number of views as output side data, the second and third data being acquired based on first data corresponding to the first number of views.

(Note 18)

A medical information processing method comprising:

acquiring, based on first data corresponding to a first number of views, second data corresponding to a second number of views and third data corresponding to a third number of views that is larger than the second number of views; and performing machine learning with the second data as input side data and the third data as output side data to generate a learned model.

(Note 19)

A medical information processing method comprising improving quality of fourth data corresponding to a fourth number of views that is smaller than the first number of views by inputting the fourth data to the learned model generated according to Note 18.

(Note 20)

An X-ray diagnostic apparatus comprising processing circuitry configured to improve quality of fourth data corresponding to a fourth number of views that is smaller than the first number of views by inputting the fourth data to the learned model generated according to Note 18.

**Claims**

1. An X-ray diagnostic apparatus (100) comprising:

   processing circuitry (200) configured to improve quality of fourth data corresponding to a fourth number of views that is smaller than a first number of views by inputting the fourth data to a learned model generated by performing machine learning with second data corresponding to a second number of views as input side data, and third data corresponding to a third number of views that is larger than the second number of views as output side data, the second data and the third data being acquired based on first data corresponding to the first number of views, wherein
   the fourth data is data acquired by tomosynthesis imaging.

2. The X-ray diagnostic apparatus (100) according to claim 1, wherein

   the first data is projection data of the first number of views, and
   the second data is generated by downsampling the first data.

3. The X-ray diagnostic apparatus (100) according to claim 1, wherein

   the first data is projection data of the first number of views, and
   the second data is generated by performing forward projection of the second number of views on reconstructed image data reconstructed from the first data.

4. The X-ray diagnostic apparatus (100)according to any one of claims 1-3, wherein

   the fourth data is projection data of the fourth number of views, and
   the processing circuitry (200) inputs the fourth data to the learned model to generate processed projection data corresponding to a fifth number of views that is larger than the fourth number of views, and performs reconstruction processing on the generated processed projection data to generate reconstructed image data.

5. The X-ray diagnostic apparatus (100) according to any one of claims 1-3, wherein

   the fourth data is reconstructed image data generated by performing reconstruction processing on projection data of the fourth number of views, and
   the processing circuitry (200) generates reconstructed image data of higher quality than quality of the fourth data by inputting the fourth data to the learned model.

6. The X-ray diagnostic apparatus (100) according to any one of claims 1-3, wherein

   the storage circuitry stores, as the learned model, a first learned model that receives input of projection data to improve quality of the projection data and a second learned model that receives input of reconstructed image data to improve quality of the reconstructed image data, and
   the processing circuitry (200) inputs projection data of the fourth number of views to the first learned model to generate processed projection data corresponding to a fifth number of views that is larger than the fourth number of views, performs reconstruction processing on the generated processed projection data to generate processed reconstructed image data, and inputs the processed reconstructed image data to the second learned model to improve quality of the processed reconstructed image data.

7. The X-ray diagnostic apparatus (100) according to any one of claims 1-3, wherein

   the fourth data is projection data of the fourth number of views, and
   the processing circuitry (200) inputs the fourth data to the learned model to adjust a parameter related to an iterative reconstruction method, and uses the adjusted parameter to generate reconstructed image data.

8. The X-ray diagnostic apparatus (100) according to claim 1, wherein the second data is data corresponding to a sparser sparse view than the third data.

9. The X-ray diagnostic apparatus (100) according to claim 1, wherein the second data is data corresponding to a limited angle with a narrower angle range than the third data.

10. The X-ray diagnostic apparatus (100) according to any one of claims 1-9, wherein the learned model is generated by machine learning using the first data as the third data.

11. The X-ray diagnostic apparatus (100) according to any one of claims 1-10, wherein the first data is X-ray data collected by computed tomography.

12. The X-ray diagnostic apparatus (100) according to any one of claims 1-10, wherein the first data and the second data are X-ray data collected by tomosynthesis imaging.

13. The X-ray diagnostic apparatus (100) according to any one of claims 1 to 12, wherein the third number of views is smaller than the first number of views.

14. The X-ray diagnostic apparatus (100) according to any one of claims 1 to 13, wherein the fourth number of views corresponds to the second number of views.

15. A medical information processing method comprising improving quality of fourth data corresponding to a fourth number of views that is smaller than a first number of views, the fourth data being data acquired by tomosynthesis imaging, by inputting the fourth data to a learned model generated by performing machine learning with second data corresponding to a second number of views as input side data, and third data corresponding to a third number of views that is larger than the second number of views as output side data, the second data and the third data being acquired based on first data corresponding to the first number of views.

# FIG.1

EP 4 167 187 A1

# FIG.2

# FIG.3

1

100
**X-RAY DIAGNOSTIC APPARATUS**

500
**STORAGE APPARATUS**

NW

400
**MEDICAL INFORMATION PROCESSING APPARATUS**

410
**PROCESSING CIRCUITRY**

411
**ACQUISITION FUNCTION**

412
**GENERATION FUNCTION**

420
**MEMORY**

FIG.4A

FIG.4B

P21

IR

I21

LEARNED MODEL M12

I22

FIG.4C

EP 4 167 187 A1

# FIG.4D

P41

LEARNED MODEL
M14

I41

## FIG.5

320

300

330

310

In training discriminator: label = 0
In training generator: label = 1

Generator
NN

fake

Discriminator
NN

input

real

In training discriminator: label = 1
not used in training generator

340

390

# FIG.6A

# FIG.6B

EP 4 167 187 A1

FIG.6C

EP 4 167 187 A1

# FIG.6D

EP 4 167 187 A1

# FIG.7

Online inferencing

Sparse-view/limited-angle sinogram → Trained GNN 300 → Corrected sinogram → Analytical reconstruction → Corrected 3D volume

500

Trained GNN 300

390

EP 4 167 187 A1

31

# FIG.8A

EP 4 167 187 A1

# FIG.8B

*c-angle is cone-angle
F-angle is fan-angle

EP 4 167 187 A1

# FIG.9

```
┌─────────────────────────┐
│  Dense-view/large-angle │
│       data(Input)       │            preprocess      ┌──────────────────┐
│                         │─────────────────────────→  │                  │
│                         │                            │                  │
└───────────┬─────────────┘                            │                  │
            │                                          │    Training      │
            │                                          │                  │
            ↓                                          │                  │
┌─────────────────────────┐                            └──────────────────┘
│ Sparse-view/limited-angle│─────────────────────────↗
│       data(Input)       │            preprocess
│                         │
│                         │
└─────────────────────────┘
```

# FIG.10

CT Data → Forward Projection Tool → Image Degradation Simulator (e.g. noise, blurring, binning)

Image Degradation Simulator → Preprocess → Simulated Dense-view/large-angle data → Training

Image Degradation Simulator → Preprocess → Simulated sparse-view/limited-angle data → Training

690

# FIG.11

Online inferencing

Sparse-view/limited-angle sinogram → Trained GNN 300 → Corrected sinogram → Analytical reconstruction → Trained GNN 600 → 3D volume

980

Dense-view/full-angel range sinogram (target)

Sparse-view/limited-angle sinogram (input)

Deep-learning model with GNN 300

3D volume from dense-view sinogram (target)

3D volume from sparse-view sinogram (input)

Deep-learning model with GNN 600

990

Offline Training

# FIG.12

Multiple dense-view/full angle range sinograms (target) → Deep-learning model

Multiple sparse-view/limited-angle sinograms (input) → Deep-learning model

Multiple Images from dense-view/full angle range acquisition (target) → Deep-learning model

Multiple Images from sparse-view/limited-angle acquisition (input) → Deep-learning model

EP 4 167 187 A1

# FIG.13

Dense-view /full angle range sinogram volume (target)

kernel

image height

Sparse-view/limited-angle sinogram volume (input)

image depth

image width

Dense-view image/full angle range volume (target)

kernel

image height

Sparse-view/ limited-angle image volume (input)

image depth

image width

EP 4 167 187 A1

Europäisches
Patentamt

European
Patent Office

Office européen
des brevets

# EUROPEAN SEARCH REPORT

Application Number

EP 22 20 1168

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LI ZIHENG ET AL: "A sinogram inpainting method based on generative adversarial network for limited-angle computed tomography", SPIE PROCEEDINGS; [PROCEEDINGS OF SPIE ISSN 0277-786X], SPIE, US, vol. 11072, 28 May 2019 (2019-05-28), pages 1107220-1107220, XP060123204, DOI: 10.1117/12.2533757 ISBN: 978-1-5106-3673-6 | 1-4,7-15 | INV. G06T11/00 |
| Y | * the whole document * | 5,6 | |
| Y | GHANI MUHAMMAD USMAN ET AL: "Integrating Data and Image Domain Deep Learning for Limited Angle Tomography using Consensus Equilibrium", 2019 IEEE/CVF INTERNATIONAL CONFERENCE ON COMPUTER VISION WORKSHOP (ICCVW), IEEE, 27 October 2019 (2019-10-27), pages 3922-3932, XP033732741, DOI: 10.1109/ICCVW.2019.00486 [retrieved on 2020-03-02] * the whole document * | 5,6 | |
| X | GHANI MUHAMMAD USMAN ET AL: "Deep Learning-Based Sinogram Completion for Low-Dose CT", 2018 IEEE 13TH IMAGE, VIDEO, AND MULTIDIMENSIONAL SIGNAL PROCESSING WORKSHOP (IVMSP), IEEE, 10 June 2018 (2018-06-10), pages 1-5, XP033394805, DOI: 10.1109/IVMSPW.2018.8448403 [retrieved on 2018-08-27] | 1,15 | TECHNICAL FIELDS SEARCHED (IPC) G06T |
| A | * figure 1 * | 2-14 | |

-/--

The present search report has been drawn up for all claims

| Place of search | Date of completion of the search | Examiner |
|---|---|---|
| Munich | 6 March 2023 | Werling, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 167 187 A1**

# EUROPEAN SEARCH REPORT

Application Number

EP 22 20 1168

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHANG YI ET AL: "Dual-domain reconstruction network for sparse-view CT", SPIE SMART STRUCTURES AND MATERIALS + NONDESTRUCTIVE EVALUATION AND HEALTH MONITORING, 2005, SAN DIEGO, CALIFORNIA, UNITED STATES, SPIE, US, vol. 11840, 9 September 2021 (2021-09-09), pages 1184016-1184016, XP060149851, ISSN: 0277-786X, DOI: 10.1117/12.2597801 ISBN: 978-1-5106-4548-6 * the whole document * | 1-15 | |

TECHNICAL FIELDS SEARCHED (IPC)

The present search report has been drawn up for all claims

| Place of search | Date of completion of the search | Examiner |
|---|---|---|
| Munich | 6 March 2023 | Werling, Alexander |

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200285902 A